# FASCICULE DE BREVET EUROPEEN

(11) **EP 2 475 694 B1**
(45) Date de publication et mention de la délivrance du brevet: **26.10.2016**
(21) Numéro de dépôt: 10740721.5
(22) Date de dépôt: 20.07.2010
(51) Int. Cl.: C08F 220/06, C08F 220/18, C08F 220/28, A61K 8/81, C08F 283/06

(54) **MONOMERES ASSOCIATIFS A BASE DE POLYCOSANOLS, EPAISSISSANTS ASSOCIATIFS CORRESPONDANTS ET LEURS UTILISATIONS**
POLYCOSANOLASSOZIATIVE MONOMERE, ZUGEHÖRIGE ASSOZIATIVE VERDICKUNGSMITTEL UND VERWENDUNGEN DAVON
POLYCOSANOL ASSOCIATIVE MONOMERS, CORRESPONDING ASSOCIATIVE THICKENERS AND USES THEREOF

(30) Priorité: 11.09.2009 FR 0904335
(43) Date de publication de la demande: 18.07.2012
(73) Titulaire: COATEX, 69730 Genay (FR)
(72) Inventeur: SUAU, Jean-Marc, F-69480 Lucenay (FR); RUHLMANN, Denis, F-69730 Genay (FR)
(74) Mandataire: Regimbeau
(86) Numéro de dépôt international: PCT/IB2010/001782
(87) Numéro de publication internationale: WO 2011/030191

(56) Documents cités:
- EP-A1- 0 013 836
- EP-A1- 0 577 525
- "Stichwort-: Wollwachs" Römpp Online 1 avril 2007 (2007-04-01), XP002577883 Extrait de l'Internet: URL:http://www.roempp.com [extrait le 2010-04-15]
- "FINAL REPORT OF THE SAFETY ASSESSEMENT FORACETYLATED LANOLIN ALCOHOL AND RELATED COMPOUNDS", , Retrieved from the Internet: URL:http://www.cir-safety.org/sites/defaul t/files/115_buff3g_suppl.pdf [retrieved on 2014-06-04]

## Description

La présente invention concerne le secteur des épaississants associatifs, et plus particulièrement des épaississants associatifs de type HASE (Emulsions modifiées hydrophobiquement et épaississantes en milieu alcalin ou Hydrophobically modified Alkali-Soluble Emulsions). Ces produits sont destinés à être mis en oeuvre dans des formulations aqueuses telles que des peintures.

L'invention ici décrite a pour principal objet de nouveaux monomères associatifs à base de polycosanols, matière première bio-ressourcée. Ils entrent dans la fabrication d'épaississants de type HASE, offrant ainsi à l'homme du métier une nouvelle gamme d'additifs rhéologiques d'origine bio-ressourcée. De plus, les épaississants ainsi fabriqués peuvent conférer aux formulations aqueuses dans lesquelles ils sont introduits des propriétés très avantageuses, comme des effets thixotropes.

Les formulations de peintures aqueuses contenant des charges minérales sont constituées d'une phase aqueuse, d'un ou plusieurs polymères en émulsion dans la phase liquide dénommés liants, de charges et/ou de pigments, d'un agent dispersant et d'adjuvants aussi divers que des tensioactifs, des agents de coalescence, des biocides, des anti-mousses et enfin, d'au moins un agent épaississant.

Ce dernier permet de maîtriser la rhéologie des formulations aqueuses dans lesquelles il est introduit, et notamment des peintures aqueuses, tant au stade de leur fabrication, que pendant leur transport, leur stockage ou au cours de leur mise en ouvre. La diversité des contraintes pratiques au niveau de chacune de ces étapes renvoie à une multiplicité de comportements rhéologiques différents. On peut néanmoins résumer le besoin de l'homme du métier à l'obtention d'un effet d'épaississement de la formulation aqueuse, tant pour des raisons de stabilité au cours du temps, que pour une possible application de la peinture sur une surface verticale, l'absence d'éclaboussures au moment de la mise en oeuvre, etc... C'est pourquoi on a désigné les additifs qui contribuent à cette régulation du comportement rhéologique sous le terme d'épaississants.

Parmi ces produits, on distingue les épaississants dits « associatifs ». qui sont des polymères hydrosolubles disposant de groupements hydrophobes insolubles. De telles macromolécules ont un caractère associant : une fois introduits dans l'eau, les groupements hydrophobes sont susceptibles de s'assembler sous forme d'agrégats micellaires. Ces agrégats sont reliés entre eux par les parties hydrophiles des polymères : il y a alors formation d'un réseau tridimensionnel qui provoque l'augmentation de la viscosité du milieu.

Le mécanisme d'action et les caractéristiques des épaississants associatifs sont aujourd'hui bien connus et décrits par exemple dans les documents « Rheology modifiers for water-borne paints » (Surface Coatings Australia, 1985, pp. 6-10) et « Rheological modifiers for water-based paints : the most flexible tools for your formulations » (Eurocoat 97, UATCM, vol. 1, pp 423-442).

Parmi ces épaississants associatifs, on distingue la classe des épaississants associatifs de type HASE (Emulsions modifiées hydrophobiquement et épaississantes en milieu alcalin selon l'acronyme anglo-saxon Hydrophobically modified Alkali-soluble Emulsions). Ils désignent des copolymères de l'acide (méth)acrylique, d'un ester de ces acides et d'un monomère dit « hydrophobe » qui est en fait constitué d'une chaîne oxyalkylée fonctionnalisable à une extrémité et terminée par un groupement hydrophobe à l'autre extrémité. Le choix du groupement hydrophobe terminal est particulièrement important, car il conditionne en grande partie les propriétés rhéologiques induites par l'épaississant correspondant.

Les documents "Dynamic light scattering of semi-dilute hydrophobically modified HASE solutions with varying length of hydrophobic alkyl chains" (Macromol. Chem. Phys., 203, 2002, pp. 2312-2321), et « Light scattering of dilute HASE solutions : effects of hydrophobicity and spacer length of macromonomer » (Macromolecules, 33, 2000, pp. 7021-7028) démontrent que la viscosité à faible gradient de cisaillement d'une peinture aqueuse contenant des épaississants de type HASE augmente, dès l'instant où le nombre de carbones dans le groupement hydrophobe est supérieur à 16.

Le document « Viscoelastic properties of HASE emulsion in salt solutions » (Polymer 40, 1999, pp. 6369-6379), qui porte sur des épaississants de type HASE dont le groupe hydrophobe est une chaîne alkyle ayant de 8 à 20 atomes de carbone, démontre que la viscosité augmente de manière très importante avec le nombre d'atomes de carbone portés par la chaîne alkyle, quel que soit le gradient de cisaillement considéré.

Aussi, l'enseignement global apporté par ces documents est que pour obtenir plus d'efficacité épaississante, une solution consiste à augmenter le nombre d'atomes de carbone portés par le groupement hydrophobe. Il s'agit là d'une des connaissances générales de l'homme du métier : pour un type de groupement hydrophobe donné, si on augmente le nombre d'atomes de carbone, on va augmenter la viscosité de la formulation aqueuse. En effet, la taille des groupements hydrophobes va conditionner la taille des agrégats micellaires qu'ils engendrent une fois en solution, ce qui est directement relié à une augmentation de la viscosité, comme le rappelle le document « Rheology modifiers for water-borne paints » (Surface Coatings Australia, 1985, pp. 6-10).

Cet enseignement est corroboré par un certain nombre de brevets qui décrivent et revendiquent, pour des polymères de type HASE, des groupement hydrophobes terminaux dont le nombre de carbone peut être égal à 30 et, dans un cas très particulier décrit ci-après supérieur à 40.

Ainsi, le document EP 0 013 836 révèle un copolymère à base d'acide méthacrylique, d'acrylate d'alkyle ayant jusqu'à 4 atomes de carbone et d'un monomère oxyalkylé terminé par une chaîne grasse ayant jusqu'à 30 atomes de carbone, capables d'augmenter sensiblement la viscosité Brookfield de gels aqueux.

Le document EP 0 011 806 décrit des polymères liquides en émulsion utilisés comme épaississants pour des compositions aqueuses -et notamment des peintures latex-résultant de la copolymérisation d'un monomère à base d'acide carboxylique, d'un monomère qui est l'acrylate d'éthyle ou ses mélanges avec un autre monomère, et enfin d'un ester tensio-actif vinylique non-ionique terminé par une chaîne alkyle ayant de 8 à 20 atomes de carbone, ou une chaîne alkyle phényle ayant de 8 à 16 atomes de carbone. Ces polymères s'avèrent efficaces à moyen et haut gradient de cisaillement, comme en témoignent respectivement les mesures de viscosité Stormer™ (moyen gradient de cisaillement) et Haake™ (haut gradient de cisaillement égal à 10 000 s⁻¹) réalisées pour ce type de polymère.

Le document EP 0 013 836 enseigne l'existence de copolymères obtenus par copolymérisation en émulsion de l'acide acrylique ou méthacrylique, d'un méthacrylate ou d'un acrylate d'alkyle, et d'un monomère non ionique terminé par un groupement alkyle, alkylaryle ou alkyle polycyclique ayant de 8 à 30 atomes de carbone. De tels additifs permettent d'améliorer le comportement au stockage des formulations aqueuses, c'est-à-dire d'augmenter la viscosité à bas gradient de cisaillement, comme l'indiquent les valeurs des viscosités Brookfield™ mesurées à 10 tours / minute.

Le document EP 0 577 526 décrit des copolymères qui sont obtenus par la copolymérisation d'un monomère éthylénique et à fonction carboxylique, d'éventuellement un monomère à insaturation éthylénique et sans fonction carboxylique, et d'un monomère oxyalkylé à insaturation éthylénique et terminé par une chaîne grasse hydrophobe, elle-même constituée de groupes linéaires ou ramifiés du type alkyle, alkylaryle, arylalkyle ou aryle ayant de 26 à 30 atomes de carbone. De tels épaississants s'avèrent particulièrement efficaces pour développer de hautes viscosités sous bas gradient de cisaillement dans des formulations aqueuses.

De même, le document EP 1 270 617 décrit des copolymères de structure identique, qui confèrent aux formulations aqueuses un profil rhéologique similaire, lesdits polymères possédant une chaîne grasse constituée de groupes linéaires ou ramifiés alkyle, alkylaryle, arylalkyle ou aryle pouvant disposer jusqu'à 36 atomes de carbone.

Parallèlement, le document EP 0 705 852 décrit un additif rhéologique qui est un copolymère obtenu par la copolymérisation d'un ou de plusieurs monomères d'acide carboxylique, d'un ou de plusieurs monomères vinyliques non ioniques, d'un ou de plusieurs monomères non ioniques contenant un groupement phénol hydrophobe substitué par un aralkyle, tel que le tristyrylphénol qui possède 30 atomes de carbone. (La Demanderesse indique que ce composé est déjà bien connu pour ses propriétés tensioactives, comme en témoigne le document WO 97/03242 qui décrit une composition permettant d'absorber les rayonnements UV, contenant notamment des di- et tristyrylphénols comme surfactants). De tels épaississants confèrent aux peintures aqueuses un bon comportement rhéologique à gradient de vitesse élevé et moyen.

Enfin, le document EP 1 812 482 décrit un procédé de fabrication de dérivés du cumyl phénol possédant plus de 40 atomes de carbone, et pour certains plus de 50 atomes de carbone : ces produits entrent dans la composition d'épaississants associatifs de type HASE au niveau du groupement hydrophobe.

Au regard de l'art antérieur, on peut chiffrer à « au moins 16 », préférentiellement « au moins 20 », très préférentiellement « au moins 24 » le nombre de carbone porté par le groupement hydrophobe, en vue d'obtenir un comportement épaississant « efficace ». A cette exigence technique, s'ajoute une contrainte d'ordre environnemental : celle de disposer aujourd'hui de produits bio-ressourcés, c'est-à-dire non issus d'une énergie fossile. Cette démarche s'inscrit dans les concepts de chimie verte et de développement durable.

A cet égard, la science n'a malheureusement pas beaucoup progressé : si la stéarine (18 atomes de carbone) a connu un certain succès, dans le cadre des groupements hydrophobes ayant plus de 24 atomes de carbone, seuls le cholestérol et la lanoline ont à notre connaissance été envisagés (respectivement 27 et 30 atomes de carbone), mais de manière anecdotique (voir page 15 du document EP 0 013 836). Par conséquent, il existe aujourd'hui une forte demande en faveur d'épaississants associatifs de type HASE, disposant d'un groupement hydrophobe terminal ayant au moins 24 atomes de carbone, et d'origine bio-ressourcée.

Poursuivant ses recherches en ce sens, la Demanderesse est parvenue à mettre au point de nouvelles structures de ce type. Elle est notamment parvenue à mettre au point de nouveaux monomères hydrophobes, qui entrent dans la synthèse d'épaississants associatifs de type HASE, lesdits monomères répondant à la formule (I) :

R - (A-O)ₘ - (B-O)ₙ - R' (I)

où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant caractérisé en ce qu'il est un groupement de formule (II) :

   CH₃-(CH₂)ₚ-CH₂- (II)
où p est un nombre réel compris entre 24 et 34, préférentiellement égal à 26 (dans ce dernier cas, R' désigne l'octocosanol).

Cette dernière formule n'est autre que celle des polycosanyls ou dérivés des polycosanols, ces derniers étant des alcools gras issus de la canne à sucre, de la cire d'abeille et de son de riz. Les polycosanols les plus répandus sont l'octocosanol, le triacontanol, le dotriacontanol, l'hexacosanol et l'heptacosanol. L'octacosanol, principal constituant, a pour formule CH₃ - (CH₂)₂₆ - CH₂ - OH. Ces produits sont aujourd'hui connus pour leur efficacité thérapeutique : propriété hypocholestérolémiante, amélioration du statut lipidique, diminution de l'agrégation plaquettaire, diminution de la prolifération des cellules musculaires, et amélioration des symptômes cardiovasculaires (voir notamment les documents EP 2 007 429, EP 1 827 136, EP 1 536 693, EP 1 776 159 et EP 1 515 714).

Rien dans l'état de la technique ne suggérait que de telles structures puissent être utilisées pour fabriquer des monomères hydrophobes, rentrant dans la composition d'épaississants associatifs de type HASE. On valorise ainsi une matière première non polluante, et aisément disponible en grande quantité.

De plus, il s'avère que lesdits épaississants conduisent à des effets rhéologiques particulièrement intéressants, lorsqu'ils sont mis en oeuvre dans des compositions aqueuses. Comme le démontrent les essais qui illustrent la présente invention, on obtient un effet d'épaississement sur une large gamme de gradient de cisaillement en fonction de la structure synthétisée : on est donc capable d'offrir une véritable gamme de produits à l'utilisateur final. Enfin, on ne détériore pas la compatibilité pigmentaire, et on est capable d'obtenir des effets de thixotropie particulièrement intéressants dans des optiques d'amélioration des compromis d'application de type tendu coulure.

On dispose donc au final d'un épaississant de type HASE particulièrement efficace au sein des compositions aqueuses dans lesquelles il est introduit, et qui dispose d'un monomère associatif terminé par un groupement hydrophobe ayant plus de 20 atomes de carbone (de 24 à 34 en réalité), ledit groupe hydrophobe étant d'origine entièrement bio-ressourcée.

Aussi, un premier objet de l'invention consiste en des monomères hydrophobes de formule (I) :

R - (A-O)ₘ - (B-O)ₙ - R' (I)

où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant caractérisé en ce qu'il est un groupement de formule (II) :

CH₃-(CH₂)ₚ-CH₂- (II)

où p est un nombre réel compris entre 24 et 34, préférentiellement égal à 26.

Un second objet de la présente invention consiste précisément en des copolymères hydrosolubles constitués :
a) d'acide (méth)acrylique,
b) d'au moins un ester de l'acide (méth)acrylique,
c) d'au moins un monomère de formule (I) :

   R - (A-O)ₘ - (B-O)ₙ - R' (I)

   où :
   - m et n sont des entiers inférieurs à 150 dont au moins un est non nul,
   - A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
   - R désigne une fonction insaturée polymérisable, préférentiellement méthacrylate,
   - R' étant caractérisé en ce qu'il est un groupement de formule (II) :

   CH₃-(CH₂)ₚ-CH₂- (II)
où p est un nombre réel compris entre 24 et 34, préférentiellement égal à 26.

La Demanderesse précise que la fabrication de ces copolymères, qui appartiennent à la famille des épaississants de type HASE, est parfaitement connue de l'homme du métier, qui pourra se reporter à l'enseignement des documents cités auparavant dans l'arrière plan technologique de la présente invention.

Ces copolymères sont aussi caractérisés en ce que ils sont constitués de, exprimé en % en poids de chacun des monomères, la somme de a), b) et c) étant égale à 100 % :
a) de 20 % à 50 %, préférentiellement de 35 % à 45 % d'acide (méth)acrylique,
b) de 40 % à 70 %, préférentiellement de 45 % à 55 % d'au moins un ester de l'acide (méth)acrylique,
c) de 2 % à 20 %, préférentiellement de 3 % à 15 % d'au moins un monomère de formule (I) :

   R - (A-O)ₘ - (B-O)ₙ - R' (I)
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant caractérisé en ce qu'il est un groupement de formule (II) :

   CH₃-(CH₂)ₚ-CH₂- (II)
où p est un nombre réel compris entre 24 et 34, préférentiellement égal à 26.

Ces copolymères sont aussi caractérisés en ce qu'ils sont obtenus par polymérisation radicalaire en solution, en émulsion directe ou inverse, en suspension ou précipitation dans des solvants, en présence de systèmes catalytiques et d'agents de transfert, ou encore en une polymérisation radicalaire contrôlée et préférentiellement en la polymérisation contrôlée par des nitroxydes (NMP) ou par des cobaloxymes, en la polymérisation par transfert d'atome radicalaire (ATRP), en la polymérisation radicalaire contrôlée par des dérivés soufrés, choisis parmi des carbamates, des dithioesters ou des trithiocarbonates (RAFT) ou des xanthates.

Ces copolymères sont caractérisés en ce qu'ils sont séparés en plusieurs phases, selon des procédés statiques ou dynamiques, par un ou plusieurs solvants polaires appartenant préférentiellement au groupe constitué par l'eau, le méthanol, l'éthanol, le propanol, l'isopropanol, les butanols, l'acétone, le tétrahydrofurane ou leurs mélanges.

Un troisième et dernier objet de la présente invention consiste en l'utilisation desdits copolymères hydrosolubles, comme agents épaississants dans une composition aqueuse, ladite composition étant préférentiellement choisie parmi une peinture aqueuse, une laque, un vernis, une sauce de couchage papetière, une formulation cosmétique ou détergente.

Les exemples qui suivent permettent de mieux comprendre l'invention, sans toutefois en limiter la portée.

### EXEMPLES

### Exemple 1

Cet exemple décrit 5 copolymères hydrosolubles, mettant en oeuvre de nouveaux monomères hydrophobes à base d'octocosanol. Ces copolymères feront l'objet de diverses utilisations dans les exemples suivants.

### Essai n° 1

Cet essai correspond à un copolymère hydrosoluble, constitué de, exprimé en % en poids de chacun de ses constituants :
a) de 36,4 % d'acide méthacrylique,
b) de 53,2% d'acrylate d'éthyle,
c) de 10,4 % d'un monomère de formule (I) :

   R - (A-O)ₘ - (B-O)ₙ - R' (I)
où m = 25, n = 0 et AO désigne l'oxyde d'éthylène, R est la fonction méthacrylate et R' désigne l'octocosanyl.

### Essai n° 2

Cet essai correspond à un copolymère hydrosoluble, constitué de, exprimé en % en poids de chacun de ses constituants :
a) de 36,4 % d'acide méthacrylique,
b) de 53,2 % d'acrylate d'éthyle,
c) de 10,4 % d'un monomère de formule (I) :

   R - (A-O)ₘ - (B-O)ₙ - R' (I)
où m = 50, n = 0 et AO désigne l'oxyde d'éthylène, R est la,fonction méthacrylate et R' désigne l'octocosanyl.

### Essai n° 3

Cet essai correspond à un copolymère hydrosoluble, constitué de, exprimé en % en poids de chacun de ses constituants :
a) de 39,3 % d'acide méthacrylique,
b) de 55,7 % d'acrylate d'éthyle,
c) de 5,0 % d'un monomère de formule (I) :

   R - (A-O)ₘ - (B-O)ₙ - R' (I)
où m = 25, n = 0 et AO désigne l'oxyde d'éthylène, R est la fonction méthacrylate et R' désigne l'octocosanyl.

### Essai n° 4

Cet essai correspond à un copolymère hydrosoluble, constitué de, exprimé en % en poids de chacun de ses constituants :
a) de 39,3 % d'acide méthacrylique,
b) de 55,7 % d'acrylate d'éthyle,
c) de 5,0 % d'un monomère de formule (I) :

   R - (A-O)ₘ - (B-O)ₙ - R' (I)
où m = 50, n = 0 et AO désigne l'oxyde d'éthylène, R est la fonction méthacrylate et R' désigne l'octocosanyl.

### Essai n° 5

Cet essai correspond à un copolymère hydrosoluble, constitué de, exprimé en % en poids de chacun de ses constituants :
a) de 41,5 % d'acide méthacrylique,
b) de 56,0 % d'acrylate d'éthyle,
c) de 2,5 % d'un monomère de formule (I) :

   R - (A-O)ₘ - (B-O)ₙ - R' (I)
où m = 25, n = 0 et AO désigne l'oxyde d'éthylène, R est la fonction méthacrylate et R' désigne l'octocosanyl (groupement de formule II avec p = 26, issu de l'octocosanol de numéro CAS = 67905-27-5).

### Essai n° 6

Cet essai correspond à un copolymère hydrosoluble, constitué de, exprimé en % en poids de chacun de ses constituants :
a) de 41,5 % d'acide méthacrylique,
b) de 56,0 % d'acrylate d'éthyle,
c) de 2,5 % d'un monomère de formule (I) :

   R - (A-O)ₘ - (B-O)ₙ - R' (I)
où m = 25, n = 0 et AO désigne l'oxyde d'éthylène, R est la fonction méthacrylate et R' désigne l'hexaconsanyl (groupement de formule II avec p = 24, issu de l'hexacosanol de numéro CAS = 506-52-5).

### Essai n° 7

Cet essai correspond à un copolymère hydrosoluble, constitué de, exprimé en % en poids de chacun de ses constituants :
a) de 41,5 % d'acide méthacrylique,
b) de 56,0 % d'acrylate d'éthyle,
c) de 2,5 % d'un monomère de formule (I) :

   R - (A-O)ₘ - (B-O)ₙ - R' (I)
où m = 25, n = 0 et AO désigne l'oxyde d'éthylène, R est la fonction méthacrylate et R' désigne le triacontanyl (groupement de formule II avec p = 24, issu du triacontanol de numéro CAS = 593-50-0).

### Essai n° 8

Cet essai correspond à un copolymère hydrosoluble, constitué de, exprimé en % en poids de chacun de ses constituants :
a) de 41,5 % d'acide méthacrylique,
b) de 56,0 % d'acrylate d'éthyle,
c) de 2,5 % d'un monomère de formule (I) :

   R - (A-O)ₘ - (B-O)ₙ - R' (I)
où m = 25, n = 0 et AO désigne l'oxyde d'éthylène, R est la fonction méthacrylate et R' désigne le dotriacontanyl (groupement de formule II avec p = 24, issu du dotriacontanol de numéro CAS = 6624-79-9).

### Exemple 2

Cet exemple illustre l'utilisation de copolymères selon l'invention et l'art antérieur, comme agents épaississant d'une peinture mate sans solvant.

La composition de ladite peinture est indiquée dans le tableau 1, les masses de chaque constituant étant indiquée en grammes. Les épaississants ont tous un extrait sec de 30 % en poids de matière active. La peinture est formulée selon les méthodes bien connues de l'homme du métier.

**Tableau 1**

| **Ingrédients de la formulation de peinture aqueuse** | **Masses (g)** |
|---|---|
| Ecodis™ P50 (dispersant COATEX™) | 4,1 |
| Tego 1488™ (anti-mousse TEGO™) | 2,3 |
| Mergal K6N (bactéricide TROY™) | 2,3 |
| TiONa RL68 (TiO₂ MILLENIUM™) | 90 |
| Omyacoat™ 850 OG (CaCO₃ OMYA™) | 223 |
| Durcal™ 5 (CaCO₃ OMYA™) | 272 |
| Mowilith™ LDM 1871 (liant CLARIANT™) | 180 |
| NaOH | 2,6 |
| Copolymère testé | 5,0 |
| Eau | 218,7 |
| **Total** | 1000 |

On détermine alors les viscosités résultantes à différents gradients de vitesse :
- à faible gradient : viscosité Brookfïeld™ à 10 et 100 tours / minute, respectivement notée µ _{Bk 10} et µ _{Bk 100} (en mPa.s),
- à moyen gradient : viscosité Stormer, notée µ _{S}
- à haut gradient : viscosité ICI, notée µ _{I}
et ce, aux instants T=0, 24 heures et 7 jours.

On rappelle que, dans le domaine des peintures aqueuses, une viscosité élevée à gradient de cisaillement élevé traduit un bon comportement « dynamique » : en pratique, la viscosité de la peinture reste suffisamment importante lors de l'étape d'application sur le support ; les bénéfices peuvent en être un garnissant (c'est-à-dire une épaisseur déposée) plus important et une propension aux éclaboussures réduite. Parallèlement, une viscosité élevée à un gradient de cisaillement faible ou moyen traduit un bon comportement « statique » : on assure ainsi une bonne stabilité au cours de leur stockage tout en évitant le phénomène de sédimentation et une limitation de la tendance à la coulure sur support vertical.

Les essais A1, A2, A3, A4 mettent respectivement en oeuvre les copolymères 1, 2, 3 et 4 selon l'invention (INV) et l'essai A6 met en oeuvre un copolymère constitué, en poids, de 55 % d'acrylate d'éthyle, 37 % d'acide méthacrylique et 8 % d'un monomère de formule (I), où m = 25, n = 0, R est la fonction méthacrylate et R' désigne une chaîne hydrocarbonée ramifiée comportant 32 atomes de carbone et issue de la pétrochimie. Les résultats apparaissent dans le tableau 2.

**Tableau 2**

| | | essai A1 INV | essai A2 INV | essai A3 INV | essai A4 INV | essai A6 AA |
|---|---|---|---|---|---|---|
| | | | | | | |
| µ_{B10} / µ_{B100} | | 7800/1000 | 8300/1200 | 10500/1400 | 10800/1500 | 14900/1900 |
| µ_{S} | T=0 | 73 | 75 | 79 | 81 | 84 |
| µ_{I} | | 0,4 | 0,4 | 0,4 | 0,5 | 0,5 |
| | | | | | | |
| µ_{B10} / µ_{B100} | | 12000/1600 | 13100/1700 | 15600/2000 | 15000/1950 | 18500/2200 |
| µ_{S} | T=24 heures | 86 | 89 | 92 | 91 | 95 |
| µ_{I} | | 0,4 | 0,4 | 0,4 | 0,5 | 0,5 |
| | | | | | | |
| µ_{B10}/µ_{B100} | | 15000/2000 | 15500/1200 | 18000/3200 | 17200/2250 | 20000/2500 |
| µ_{S} | T=7 jours | 92 | 93 | 96 | 95 | 98 |
| µ_{I} | | 0,4 | 0,4 | 0,4 | 0,5 | 0,5 |

Ce tableau démontre que les copolymères selon l'invention permettent d'épaissir efficacement une peinture mate sans solvant, quel que soit le gradient de cisaillement. Si les viscosités obtenues se situent au niveau de celles mesurées pour la référence, les variations observées en fonction des structures synthétisées démontrent la possibilité de développer une gamme d'épaississants à partir de tels copolymères. Ces derniers constituent donc une alternative efficace en terme de performances et qui présentent l'avantage de disposer d'un groupe hydrophobe d'origine bio-ressourcée.

### Exemple 3

Cet exemple illustre l'utilisation de copolymères selon l'invention et l'art antérieur, comme agents épaississant d'une peinture satinée avec solvant.

La composition de ladite peinture est indiquée dans le tableau 3, les masses de chaque constituant étant indiquée en grammes. Les épaississants ont tous un extrait sec de 30 % en poids de matière active. La peinture est formulée selon les méthodes bien connues de l'homme du métier.

**Tableau 3**

| **Ingrédients de la formulation de peinture aqueuse** | **Masses (g)** |
|---|---|
| Coadis™ BR3 (dispersant COATEX™) | 3,5 |
| Tego 1488™ (anti-mousse TEGO™) | 2,0 |
| Mergal K6N (bactéricide TROY™) | 2,0 |
| TiONa RHD2 (TiO₂ HUNTSMAN™) | 190 |
| Omyacoat™ 850 OG (CaCO₃ OMYA™) | 130 |
| Texanol™ (agent coalescence EASTMAN CHEMICALS™) | 20,0 |
| Acronal™ 290 D (liant BASF™) | 424 |
| NaOH | 5,1 |
| Monoéthylène glycol | 20,0 |
| Copolymère testé | 5,0 |
| Eau | 198,4 |
| **Total** | 1000 |

Les essais B1, B3, B5, B6, B7 et B8 mettent respectivement en ouvre les copolymères 1, 3 et 5, 6, 7 et 8 selon l'invention (INV) et l'essai B6 met en oeuvre le même copolymère de l'art antérieur (AA) que pour l'essai A6. Les résultats apparaissent dans le tableau 4.

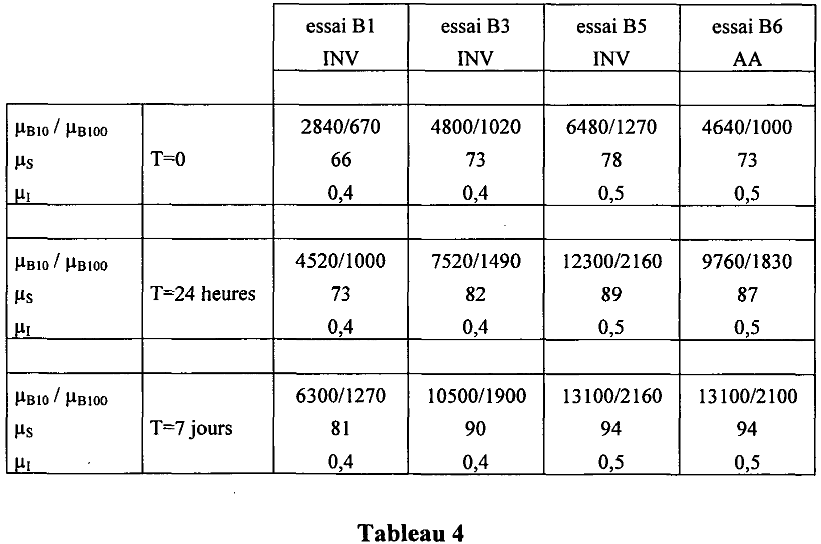

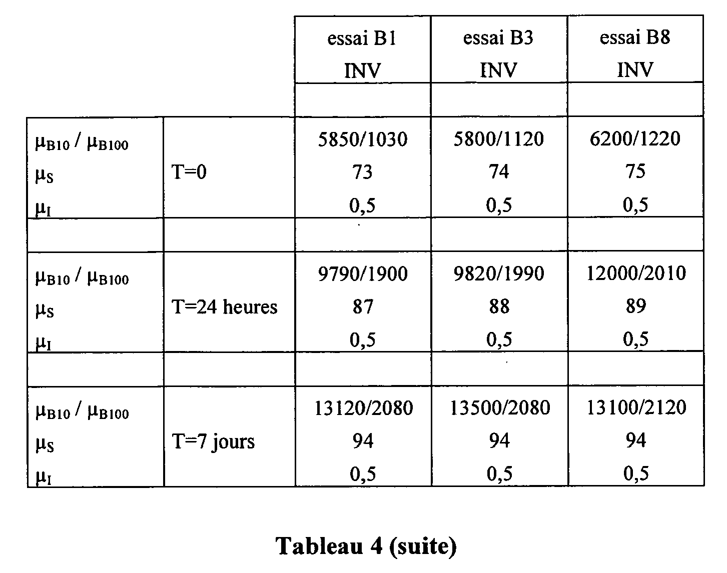

Ce tableau démontre que les copolymères selon l'invention permettent d'épaissir efficacement une peinture mate sans solvant, quel que soit le gradient de cisaillement. Les essais B5 à B8 permettent même d'obtenir des viscosités supérieures à celles mesurées selon l'art antérieur B6 et ce, pour seulement 2,5 % en poids de monomère associatif contre 10 % pour le produit de l'art antérieur.
En outre, les variations observées en fonction des structures synthétisées démontrent la possibilité de développer une gamme d'épaississants à partir de tels copolymères. Ces derniers constituent donc une alternative efficace en terme de performances et qui présentent l'avantage de disposer d'un groupe hydrophobe d'origine bio-ressourcée.

### Exemple 4

Cet exemple illustre l'utilisation de copolymères selon l'invention et l'art antérieur, comme agents épaississants d'une lasure.

La composition de ladite lasure est indiquée dans le tableau 5. Les épaississants ont tous un extrait sec de 30 % en poids de matière active. La peinture est formulée selon les méthodes bien connues de l'homme du métier.

**Tableau 5**

| **Ingrédients de la formulation de peinture aqueuse** | **Masses (g)** |
|---|---|
| Nopco™ (anti-mousse COGNIS™) | 1,3 |
| Mergal K6N (bactéricide TROY™) | 17,1 |
| Acematt TS 100™ (silice DEGUSSA ™) | 2,4 |
| Acronal™ LR 9014 (liant BASF™) | 337,38 |
| Luconyl™ noir (pigment BASF™) | 0,06 |
| Luconyl™ rouge (pigment BASF™) | 0,3 |
| Luconyl™ noir (pigment BASF™) | 12,06 |
| NH₄OH | 2,25 |
| Copolymère testé | 5 |
| Eau | 222,15 |
| **Total** | 600 |

L'essai C3 met en ouvre le copolymère 3 selon l'invention (INV) et l'essai C6 met en oeuvre le même produit de l'art antérieur (AA) que l'essai A6. Les résultats apparaissent dans le tableau 6.

**Tableau 6**

| | | essai C3 INV | essai C6 AA |
|---|---|---|---|
| | | | |
| µ_{B10} / µ_{B100} | | 3360/870 | 3150/820 |
| µ_{S} | T=0 | 69 | 69 |
| µ_{I} | | 0,3 | 0,3 |
| | | | |
| µ_{B10} / µ_{B100} | | 13700/2680 | 14700/2690 |
| µ_{S} | T=24 heures | 98 | 98 |
| µ_{I} | | 0,4 | 0,4 |

Ce tableau démontre que le copolymère selon l'invention permet d'épaissir efficacement une lasure, quel que soit le gradient de cisaillement. Les performances épaississantes se situent au même niveau que la référence de l'art antérieur.

De plus, on a réalisé 2 tests de compatibilité pigmentaire. En pratique, si une peinture présente une compatibilité pigmentaire insuffisante, on observe d'une part une chute de la viscosité et d'autre part une faible force colorante nécessitant une quantité plus importante de colorants pour obtenir un certain niveau de teinte. On obtient alors un film de peinture de teinte trop claire et manquant d'uniformité par rapport à la référence. Ce phénomène peut être mesuré par l'utilisation d'un spectrophotocolorimètre permettant de mesurer les coordonnées tri-chromatiques (Huntsmann : L*,a*,b* ) donc la couleur d'un film de peinture sec.

On réalise aussi un « essai de gommage au doigt », connu par l'homme du métier sous le vocable « rub out ». Il consiste à appliquer sans cisaillement, à l'aide d'un filmographe, 150 µm de la formulation de peinture sur une carte de contraste, c'est-à-dire lentement et sans contrainte et à attendre 45 secondes puis à appliquer un cisaillement par frottement, avec le doigt, du film encore visqueux de peinture, pendant 30 secondes en un endroit quelconque. Après séchage du film, la différence colorimétrique entre la zone cisaillée (zone frottée) et la zone non cisaillée (zone du film non frotté) déterminée au moyen d'un spectro-colorimètre Spectro-pen, permet d'évaluer (valeur du ΔE) si la composition de peinture testée présente ou non une bonne compatibilité pigmentaire.

Ces 2 derniers tests sont réalisés sur une peinture dans laquelle on a ajouté 5 % en poids d'un pigment noir qui est la Laconyl™ noir (BASF™).

**Tableau 7**

| | | essai C3 INV | essai C6 AA |
|---|---|---|---|
| | | | |
| µB₁₀ / µB₁₀₀ | T=0 | 16200/2680 | 27500/3700 |
| µ_{S} | après introduction | 94 | 104 |
| µ_{I} | du pigment | 0,4 | 0,4 |
| | | | |
| µ_{B10} / µ_{B100} | T=24 heures | 4280/1700 | 4800/1400 |
| µ_{S} | après introduction | 75 | 82 |
| µ_{I} | du pigment | 0,3 | 0,3 |
| | | | |
| ΔE | | 0,8 | 0,8 |
| L* fond noir | | 10,0 | 10,0 |
| L* fond blanc | | 10,0 | 10,0 |

Si les valeurs de ΔE et de L* ne sont pas affectées, la chute de viscosité à bas et moyen gradient de vitesse est plus importante dans le cadre de l'art antérieur, ce qui démontre une meilleure compatibilité pigmentaire avec l'invention.

### Exemple 5

Cet exemple illustre l'utilisation de copolymères selon l'invention et l'art antérieur, comme agents épaississants de la même lasure que celle utilisée dans l'exemple 4.
On évalue ici le caractère thixotrope des profils rhéologiques observés, dans le cas d'un épaississant de l'art antérieur qui est le polymère selon l'essai A6 (ou C6), d'un épaississant de l'invention qui est le polymère selon l'essai C5, et d'une référence qui est un épaississant non thixotrope commercialisé par la société COATEX™ sous le nom Viscoatex 730.

D'un point de vue expérimental, le test consiste à appliquer, au moyen d'un rhéomètre Rheostress™ RS 150 commercialisé par la société HAAKE™, un cycle de cisaillement correspondant à une augmentation puis à une diminution du cisaillement. On établit alors un rhéogramme qui est la courbe traduisant l'évolution de la viscosité en fonction du cisaillement. Si un produit est thixotrope, le chemin tracé au retour se situe nettement en dessous de celui établi à l'aller. L'amplitude de la thixtropie est quantifiée par la surface de l'aire déterminée par les courbes aller et retour. Un comportement thixotrope facilite notamment une application des peintures sur des surfaces verticales.

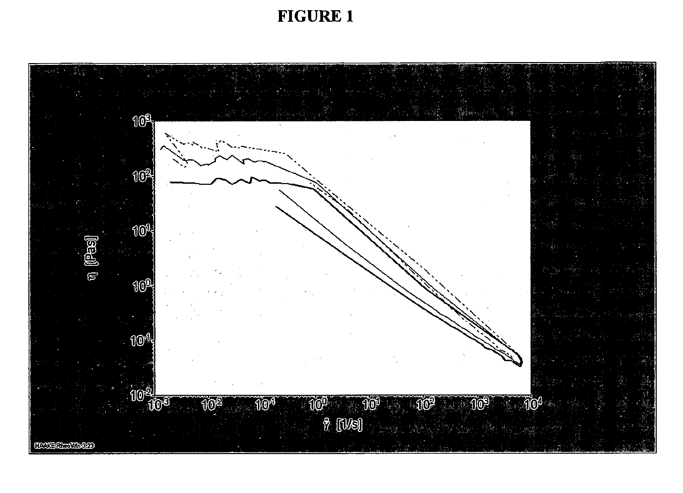

La figure 1 / 1 représente les rhéogrammes obtenus pour un épaississant de l'art antérieur non thixotrope qui est le Viscoatex 730 (tracé gris en pointillés), pour le polymère de l'art antérieur A6 (tracé gris) et pour le polymère selon l'invention C5 (tracé noir).
Or, ce dernier présente un comportement thixotrope au moins aussi marqué que pour le produit de l'art antérieur (dont les extrémités hydrophobes sont issues d'une énergie fossile) : il pourra donc être utilisé de manière très avantageuse pour épaissir une peinture en vue d'une application sur une surface verticale.

## Revendications

1. - Monomères hydrophobes de formule (I) :
R - (A-O)ₘ - (B-O)ₙ - R' (I)
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant **caractérisé en ce qu'**il est un groupement de formule (II) :
CH₃ - (CH₂)ₚ - CH₂ - (II)
où p est un nombre réel compris entre 24 et 34, préférentiellement égal à 26.

2. - Copolymères hydrosolubles constitués :
a) d'acide (méth)acrylique,
b) d'au moins un ester de l'acide (méth)acrylique,
c) d'au moins un monomère de formule (I) :
R - (A-O)ₘ - (B-O)ₙ - R' (I)
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant **caractérisé en ce qu'**il est un groupement de formule (II) :
CH₃ - (CH₂)ₚ - CH₂ - (II)
où p est un nombre réel compris entre 24 et 34, préférentiellement égal à 26.

3. - Copolymères selon la revendication 2, **caractérisés en ce que** ils sont constitués de, exprimé en % en poids de chacun des monomères, la somme de a), b) et c) étant égale à 100% :
a) de 20% à 50%, préférentiellement de 35% à 45% d'acide (méth)acrylique,
b) de 40% à 70%, préférentiellement de 45% à 55% d'au moins un ester de l'acide (méth)acrylique,
c) de 2% à 20%, préférentiellement de 3% à 15% d'au moins un monomère de formule (I) :
R - (A-O)ₘ - (B-O)ₙ - R' (I)
où :
- m et n sont des entiers inférieurs à 150 dont au moins un est non nul,
- A et B désignent des groupes alkyles différents l'un de l'autre, et ayant de 2 à 4 atomes de carbone, le groupement AO désignant préférentiellement l'oxyde d'éthylène et le groupement BO désignant préférentiellement l'oxyde de propylène,
- R désigne une fonction insaturée polymérisable, préférentiellement méthacrylate,
- R' étant **caractérisé en ce qu'**il est un groupement de formule (II) :
CH₃ - (CH₂)ₚ - CH₂ - (II)
où p est un nombre réel compris entre 24 et 34, préférentiellement égal à 26.

4. - Copolymères selon l'une des revendications 2 ou 3, **caractérisés en ce qu'**ils sont obtenus par polymérisation radicalaire en solution, en émulsion directe ou inverse, en suspension ou précipitation dans des solvants, en présence de systèmes catalytiques et d'agents de transfert, ou encore en une polymérisation radicalaire contrôlée et préférentiellement en la polymérisation contrôlée par des nitroxydes (NMP) ou par des cobaloxymes, en la polymérisation par transfert d'atome radicalaire (ATRP), en la polymérisation radicalaire contrôlée par des dérivés soufrés, choisis parmi des carbamates, des dithioesters ou des trithiocarbonates (RAFT) ou des xanthates.

5. - Copolymères selon l'une des revendications 2 à 4, **caractérisés en ce qu'**ils sont séparés en plusieurs phases, selon des procédés statiques ou dynamiques, par un ou plusieurs solvants polaires appartenant préférentiellement au groupe constitué par l'eau, le méthanol, l'éthanol, le propanol, l'isopropanol, les butanols, l'acétone, le tétrahydrofurane ou leurs mélanges.

6. - Utilisation des copolymères selon l'une des revendications 2 à 5, comme agents épaississants dans une composition aqueuse, ladite composition étant préférentiellement choisie parmi une peinture aqueuse, une laque, un vernis, une sauce de couchage papetière, une formulation cosmétique ou détergente.

## Patentansprüche

1. - Hydrophobe Monomere der Formel (I):
R - (A-O)ₘ - (B-O)ₙ - R' (I)
wobei:
- m und n ganze Zahlen sind, die kleiner als 150 sind, wobei mindestens eine davon von Null verschieden ist,
- A und B Alkylgruppen bezeichnen, die sich voneinander unterscheiden und 2 bis 4 Kohlenstoffatome aufweisen, wobei die Gruppe AO vorzugsweise Ethylenoxid bezeichnet und die Gruppe BO vorzugsweise Propylenoxid bezeichnet,
- R eine ungesättigte polymerisierbare funktionelle Gruppe bezeichnet, vorzugsweise Methacrylat,
- R' **dadurch gekennzeichnet ist, dass** es sich um eine Gruppe der Formel (II) handelt:
CH₃ - (CH₂)ₚ - CH₂ - (II)
wobei p eine reelle Zahl im Bereich von 24 bis 34 ist, wobei sie vorzugsweise gleich 26 ist.

2. - Wasserlösliche Copolymere, die aus Folgendem bestehen:
a) (Meth)acrylsäure,
b) mindestens einem (Meth)acrylsäureester,
c) mindestens einem Monomer der Formel (I):
R - (A-O)ₘ - (B-O)ₙ - R' (I)
wobei:
- m und n ganze Zahlen sind, die kleiner als 150 sind, wobei mindestens eine davon von Null verschieden ist,
- A und B Alkylgruppen bezeichnen, die sich voneinander unterscheiden und 2 bis 4 Kohlenstoffatome aufweisen, wobei die Gruppe AO vorzugsweise Ethylenoxid bezeichnet und die Gruppe BO vorzugsweise Propylenoxid bezeichnet,
- R eine ungesättigte polymerisierbare funktionelle Gruppe bezeichnet, vorzugsweise Methacrylat,
- R' **dadurch gekennzeichnet ist, dass** es sich um eine Gruppe der Formel (II) handelt:
CH₃ - (CH₂)ₚ - CH₂ - (II)
wobei p eine reelle Zahl im Bereich von 24 bis 34 ist, wobei sie vorzugsweise gleich 26 ist.

3. - Copolymere nach Anspruch 2, **dadurch gekennzeichnet, dass** sie, ausgedrückt in Gewichts-% jedes der Monomere, aus Folgendem bestehen, wobei die Summe von a), b) und c) gleich 100 % ist:
a) 20 % bis 50 %, vorzugsweise 35 % bis 45 % an (Meth)acrylsäure,
b) 40 % bis 70 %, vorzugsweise 45 % bis 55 %, mindestens eines (Meth)acrylsäureesters,
c) 2 % bis 20 %, vorzugsweise 3 % bis 15 %, mindestens eines Monomers der Formel (I):
R - (A-O)ₘ - (B-O)ₙ - R' (I)
wobei:
- m und n ganze Zahlen sind, die kleiner als 150 sind, wobei mindestens eine davon von Null verschieden ist,
- A und B Alkylgruppen bezeichnen, die sich voneinander unterscheiden und 2 bis 4 Kohlenstoffatome aufweisen, wobei die Gruppe AO vorzugsweise Ethylenoxid bezeichnet und die Gruppe BO vorzugsweise Propylenoxid bezeichnet,
- R eine ungesättigte polymerisierbare funktionelle Gruppe bezeichnet, vorzugsweise Methacrylat,
- R' **dadurch gekennzeichnet ist, dass** es sich um eine Gruppe der Formel (II) handelt:
CH₃ - (CH₂)ₚ - CH₂ - (II)
wobei p eine reelle Zahl im Bereich von 24 bis 34 ist, wobei sie vorzugsweise gleich 26 ist.

4. - Copolymere nach einem der Ansprüche 2 oder 3, **dadurch gekennzeichnet, dass** sie durch radikalische Polymerisation in Lösung, in direkter oder umgekehrter Emulsion, in Suspension oder mit Fällungsvorgang in Lösungsmitteln, in Gegenwert von katalytischen Systemen und Kettenübertragungsmitteln erhalten werden, oder auch in einer kontrollierten radikalischen Polymerisation, und zwar vorzugsweise in einer solchen Polymerisation, die mittels Nitroxiden (NMP) oder mittels Cobaloximen kontrolliert wird, in einer solchen radikalischen Polymerisation unter Atomtransfer (ATRP), in einer solchen radikalischen Polymerisation, die mittels schwefelhaltiger Verbindungen, welche aus den Carbamaten, den Dithioestern oder den Trithiocarbonaten (RAFT) oder den Xanthogenaten ausgewählt sind, kontrolliert wird.

5. - Copolymere nach einem der Ansprüche 2 bis 4, **dadurch gekennzeichnet, dass** sie in mehrere Phasen aufgetrennt werden, und zwar gemäß statischen oder dynamischen Verfahren mittels eines oder mehrerer polarer Lösungsmittel, die vorzugsweise der Gruppe angehören, welche aus Wasser, Methanol, Ethanol, Propanol, Isopropanol, den Butanolen, Aceton, Tetrahydrofuran oder deren Mischungen besteht.

6. - Verwendung der Copolymere nach einem der Ansprüche 2 bis 5 als Verdickungsmittel in einer wässrigen Zusammensetzung, wobei die Zusammensetzung vorzugsweise aus einem wässrigen Anstrichmittel, einem Farblack, einem Klarlack, einer Papierstreichmasse, einer kosmetischen oder reinigenden Formulierung ausgewählt ist.

## Claims

1. - Hydrophobic monomers of formula (I):
R - (A-O)m - (B-O)ₙ - R' (I)
where:
- m and n are integers of less than 150, at least one of which is non-zero,
- A and B designate alkyl groups different from one another, and having 2 to 4 carbon atoms, wherein group AO preferentially designates ethylene oxide, and group BO preferentially designates propylene oxide,
- R designates a polymerisable unsaturated group, preferentially methacrylate,
- where R' is **characterised in that** it is a grouping of formula (II):
CH₃ - (CH₂)ₚ - CH₂ - (II)
where p is a real number comprised between 24 and 34, preferentially equal to 26.

2. - Hydrosoluble copolymers consisting of:
a) (meth)acrylic acid,
b) at least one ester of (meth)acrylic acid,
c) of at least one monomer of formula (I):
R - (A-O)m - (B-O)ₙ - R' (I)
where:
- m and n are integers of less than 150, at least one of which is non-zero,
- A and B designate alkyl groups different from one another, and having 2 to 4 carbon atoms, wherein group AO preferentially designates ethylene oxide, and group BO preferentially designates propylene oxide,
- R designates a polymerisable unsaturated group, preferentially methacrylate,
- where R' is **characterised in that** it is a grouping of formula (II):
CH₃ - (CH₂)ₚ - CH₂ - (II)
where p is a real number comprised between 24 and 34, preferentially equal to 26.

3. - Copolymers according to claim 2, **characterised in that** they consist, expressed as a % by weight of each of the monomers, wherein the sum of a), b) and c) is equal to 100%, of:
a) from 20% to 50%, preferentially from 35% to 45%, of (meth)acrylic acid,
b) from 40% to 70%, preferentially from 45% to 55%, of at least one ester of (meth)acrylic acid,
c) from 2% to 20%, preferentially from 3% to 15%, of at least one monomer of formula (I):
R - (A-O)m - (B-O)ₙ - R' (I)
where:
- m and n are integers of less than 150, at least one of which is non-zero,
- A and B designate alkyl groups different from one another, and having 2 to 4 carbon atoms, where group AO preferentially designates ethylene oxide, and group BO preferentially designates propylene oxide,
- R designates a polymerisable unsaturated group, preferentially methacrylate,
- wherein R' is **characterised in that** it is a grouping of formula (II):
CH₃ - (CH₂)ₚ - CH₂ - (II)
where p is a real number comprised between 24 and 34, preferentially equal to 26.

4. - Copolymers according to one of claims 2 or 3, **characterised in that** they are obtained by radical polymerisation in solution, in a direct or reverse emulsion, in suspension or precipitation in solvents, in the presence of catalytic systems and transfer agents, or again under controlled radical Polymerisation, and preferentially under nitroxide mediated polymerisation (NMP) or by cobaloximes, under atom transfer radical polymerisation (ATRP), under controlled radical polymerisation by sulphurated derivatives, chosen from among carbamates, dithioesters or trithiocarbonates (RAFT) or xanthates.

5. - Copolymers according to one of claims 2 to 4, **characterised in that** the said copolymers may be, treated and separated into several phases, according to static or dynamic processes, by one or more polar solvents belonging preferentially to the group constituted by water, methanol, ethanol, propanol, isopropanol, the butanols, acetone, tetrahydrofuran or their mixtures thereof.

6. - Use of copolymers according to one of claims 2 to 5, as thickening agents in an aqueous composition, wherein the said composition is preferentially chosen from among an aqueous paint, a lacquer, a varnish, a paper coating, or a cosmetic or detergent formulation.
